# FASCICULE DE BREVET EUROPEEN

(11) **EP 2 921 168 B1**
(45) Date de publication et mention de la délivrance du brevet: **13.04.2016**
(21) Numéro de dépôt: 15162899.7
(22) Date de dépôt: 14.06.2011
(51) Int. Cl.: A61K 9/20, A61K 31/4045, A61K 31/55, A61K 45/06, A61P 9/04

(54) **ASSOCIATION DE LA N-{[(7S)-3,4-DIMETHOXYBICYCLO[4.2.0]OCTA-1,3,5-TRIEN-7-YL]METHYL}-3-(7,8-DIMETHOXY-1,2,4,5-TETRAHYDRO-3H-3-BENZAZEPIN-3-YL)-N-METHYL-3-OXO-1-PROPANAMINE ET DU PERINDOPRIL**
ASSOZIATION VON N-{[(7S)-3,4-DIMETHOXYBICYCLO[4.2.0]OCTA-1,3,5-TRIEN-7-YL]METHYL}-3-(7,8-DIMETHOXY-1,2,4,5-TETRAHYDRO-3H-3-BENZAZEPIN-3-YL)-N-METHYL-3-OXO-1-PROPANAMIN UND PERINDOPRIL
ASSOCIATION OF N-{[(7S)-3,4-DIMETHOXYBICYCLO[4.2.0]OCTA-1,3,5-TRIEN-7-YL]METHYL}-3-(7,8-DIMETHOXY-1,2,4,5-TETRAHYDRO-3H-3-BENZAZEPIN-3-YL)-N-METHYL-3-OXO-1-PROPANAMINE AND PERINDOPRIL

(30) Priorité: 15.06.2010 FR 1002525
(43) Date de publication de la demande: 23.09.2015
(62) Demande divisionnaire de: 11290269.7
(73) Titulaire: Les Laboratoires Servier, 92284 Suresnes Cedex (FR)
(72) Inventeur: THUILLEZ, Christian, 76000 ROUEN (FR); MULDER, Paulus, 76130 MONT SAINT AIGNAN (FR); VILAINE, Jean-Paul, 92290 CHATENAY MALABRY (FR); FRATACCI, Marie-Dominique, 78390 BOIS D'ARCY (FR); LEREBOURS-PIGEONNIERE, Guy, 92300 LEVALLOIS-PERRET (FR); FELDMANN, Luc, 75017 PARIS (FR); ROUSSEL, Jérôme, 78220 VIROFLAY (FR)

(56) Documents cités:
- EP-A1- 1 354 873
- EP-A1- 1 362 590
- EP-A1- 2 036 892
- EP-A1- 2 039 682
- RU-C1- 2 364 401

## Description

La présente invention concerne l'association de la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N-*méthyl-3-oxo-1-propanamine de formule (II), 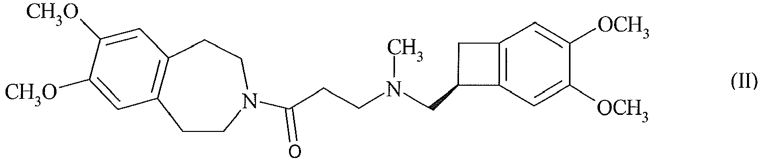 ainsi que ses sels d'addition à un acide pharmaceutiquement acceptable, leurs hydrates et formes cristallines, et du perindopril.

Cette association est utile pour l'obtention de médicaments destinés au traitement de l'insuffisance cardiaque, plus particulièrement de l'insuffisance cardiaque à fonction systolique conservée.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, pamoïque, 1,5-naphtalènedisulfonique.

Les inhibiteurs sélectifs et spécifiques du courant I_{f} sinusal et plus particulièrement la *N-*{[(7*S*)-3,4-diméthoxybicyclo[4.2.0]octa-1,3,5-trién-7-yl]méthyl}-3-(7,8-diméthoxy-1,2,4,5-tétrahydro-3*H-*3-benzazépin-3-yl)-*N*-méthyl-3-oxo-1-propanamine, ainsi que ses sels d'addition à un acide pharmaceutiquement acceptable, et plus particulièrement son chlorhydrate et son fumarate, leurs hydrates et formes cristallines, possèdent des propriétés pharmacologiques et thérapeutiques très intéressantes, notamment des propriétés chronotropes négatives (réduction de la fréquence cardiaque), qui rendent ces composés utiles dans le traitement, la prévention et l'amélioration du pronostic de différentes maladies cardio-vasculaires liées à l'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque chronique.

La préparation et l'utilisation en thérapeutique de la *N*-{[(7*S*)-3,4-diméthoxybicyclo[4.2.0]octa-1,3,5-trién-7-yl]méthyl}-3-(7,8-diméthoxy-1,2,4,5-tétrahydro -3*H*-3-benzazépin-3-yl)-*N*-méthyl-3-oxo-1-propanamine et de ses sels d'addition à un acide pharmaceutiquement acceptable, et plus particulièrement de son chlorhydrate et de son fumarate, ont été décrits dans le brevet européen EP 2 036 892.

Le perindopril est un inhibiteur de l'enzyme de conversion de l'angiotensine.
Les inhibiteurs de l'enzyme de conversion de l'angiotensine sont une des classes thérapeutiques majeures dans le traitement de l'hypertension artérielle. Ils agissent principalement par l'inhibition de la synthèse de l'angiotensine II et par un blocage de la dégradation de la bradykinine.
Ils ont montré qu'au delà de la baisse de la pression artérielle ils amélioraient la morbidité (infarctus du myocarde, accidents vasculaires cérébraux) et la mortalité cardiovasculaire des hypertendus, des diabétiques, des malades avec une maladie coronaire préexistante.

La demanderesse a découvert que l'association de la N-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine, et du perindopril, possédait des propriétés intéressantes permettant son utilisation dans le traitement de l'insuffisance cardiaque, plus particulièrement de l'insuffisance cardiaque à fonction systolique conservée.

L'insuffisance cardiaque par dysfonction systolique du ventricule gauche n'est plus la seule forme d'insuffisance cardiaque. De plus en plus souvent, les patients qui ont une insuffisance cardiaque ont une fraction d'éjection supérieure à 40 %. La proportion d'insuffisance cardiaque dite « diastolique » (ou plutôt « à fonction systolique conservée ») augmente avec l'âge. Elle rend compte actuellement de 30 à 40 % des hospitalisations pour insuffisance cardiaque et, après 80 ans, dépasse en fréquence celle des insuffisances cardiaques par dysfonction systolique. Les insuffisances cardiaques diastoliques associent généralement une prolongation de la relaxation ventriculaire et une réduction de la distensibilité de la chambre ventriculaire gauche. Les causes essentielles sont les cardiopathies ischémiques, hypertensives et du sujet âgé. Les facteurs prédisposant sont l'age, le sexe (femme), le diabète, l'obésité et l'hypertension artérielle. Le remodelage concentrique du ventricule gauche, avec ou sans hypertrophie, entraîne constamment un trouble de la fonction diastolique. On trouve le plus souvent un facteur déclenchant à l'origine d'une poussée congestive. L'insuffisance cardiaque dite diastolique verra sa fréquence croître avec l'âge. Sa physiopathologie reste complexe et justifie d'être mieux comprise par les cliniciens.

Aucun traitement n'a aujourd'hui démontré d'efficacité dans cette pathologie, dont la mortalité, de 50% à 4 ans, est équivalente à celle de l'insuffisance cardiaque systolique.

La demanderesse a découvert que l'utilisation de l'association de la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine et du perindopril permettait d'obtenir des effets pharmacologiques supérieurs à ceux observés en utilisant soit la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine seule, soit le perindopril seul. De plus, l'utilisation de l'association de la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine et du perindopril permet de ramener les paramètres physiologiques observés à des valeurs très proches de la normale. Ces observations permettent d'envisager l'utilisation de l'association de la *N-*{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine et du perindopril dans le traitement de l'insuffisance cardiaque, plus particulièrement de l'insuffisance cardiaque à fonction systolique conservée.

La N{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine est préférentiellement utilisée sous forme de chlorhydrate ou de fumarate ou de l'un de leurs hydrates ou formes cristallines.

Le perindopril peut être utilisé sous forme de ses sels d'addition à un acide ou à une base pharmaceutiquement acceptable, leurs hydrates et leurs formes cristallines, plus particulièrement ses sels de *tert*-butylamine ou d'arginine, leurs hydrates et formes cristallines.

La présente invention s'étend plus particulièrement à l'association de la *N*-{[(7*S*)-3,4-diméthoxybicyclo[4.2.0]octa-1,3,5-trién-7-yl]méthyl}-3-(7,8-diméthoxy-1,2,4,5-tétrahydro -3*H-*3-benzazépin-3-yl)-*N-*méthyl-3-oxo-1-propanamine ou l'un de ses sels d'addition à un acide pharmaceutiquement acceptable, leurs hydrates ou formes cristallines, et du perindopril ou un de ses sels d'addition à une base pharmaceutiquement acceptable, et plus particulièrement ses sels de *tert*-butylamine ou d'arginine, leurs hydrates ou formes cristallines.

La posologie utile varie selon le sexe, l'âge et le poids du patient, la voie d'administration, la nature de l'affection et des traitements éventuellement associés.

La dose de *N*-{[(7S)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-N-méthyl-3-oxo-1-propanamine (ci après dénommé composé A) pourra varier de 5 à 100 mg par jour.

La dose journalière de perindopril sera préférentiellement comprise entre 1 et 10 mg inclus. Les compositions pharmaceutiques pouvant être utilisés sont celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc... ainsi que les compositions pharmaceutiques avec libération programmée, retardée, prolongée ou différée.

Outre la *N*-{[(7S)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-N-méthyl-3-oxo-1-propanamine et le perindopril, lesdites compositions pharmaceutiques contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants :* l'agar, l'acide alginique et son sel de sodium, les mélanges effervescents.

Les exemples suivants illustrent l'invention.

**Liste des abréviations utilisées**

| | |
|---|---|
| dP/dtₘₐₓ | : augmentation maximale de pression par seconde |
| dP/dtₘᵢₙ | : diminution maximale de pression par seconde |
| IC | : insuffisance cardiaque |
| LVEDP | : Left Ventricular End Diastolic Pressure (pression télé-diastolique du ventricule gauche) |
| LVEDPVR | : Left Ventricular End Diastolic Pressure Volume Relation (relation pression-volume télé-diastolique du ventricule gauche) |
| LVESP | : Left Ventricular End Systolic Pressure (pression télé-systolique du ventricule gauche) |
| LVESPVR | : Left Ventricular End Systolic Pressure Volume Relation (relation pression-volume télé-systolique du ventricule gauche) |
| VG | : ventricule gauche |

### Tests pharmacologiques :

Une insuffisance cardiaque est provoquée chez des rats en pratiquant une ligature de l'artère coronaire gauche (les animaux témoins subissent une opération mais ne sont pas ligaturés) qui provoque une ischémie d'une partie de la paroi du ventricule gauche. Les animaux récupèrent pendant 7 jours puis, pendant 12 semaines, reçoivent soit 3 mg/kg du composé A, soit 0.4 mg/kg de perindopril, soit concomitamment perindopril et le composé A.

Douze semaines après l'opération, on constate que les animaux ayant subi la ligature coronaire développent une insuffisance cardiaque à la fois systolique (anomalie de l'éjection) et diastolique (anomalie du remplissage).

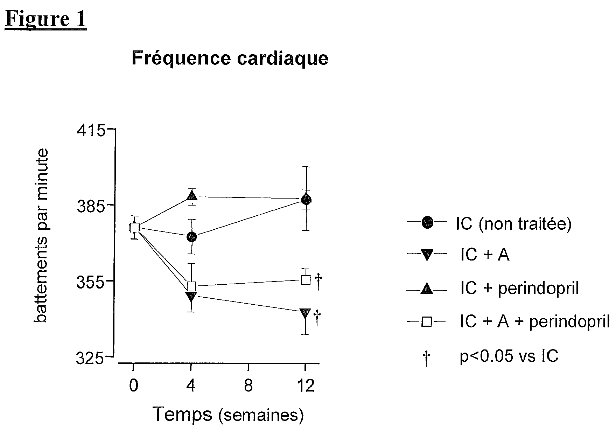

Chez ces animaux, le composé A permet de diminuer significativement la fréquence cardiaque, seul ou associé à perindopril (tableau 1 et figure 1).

**Tableau 1**

| | | | **IC (non traitée)** | **IC + A** | **IC + perindopril** | **IC + A + perindopril** |
|---|---|---|---|---|---|---|
| **Fréquence cardiaque** (bpm) | durée de traitement | 4 semaines | 372,5 | 349,3 | 388,2 | 352,8 |
| | | 12 semaines | 387,2 | 342,7^{†} | 387,7 | 353,1^{†} |

| | | | | | | |
|---|---|---|---|---|---|---|
| ^{†}p<0,05 vs IC | | | | | | |

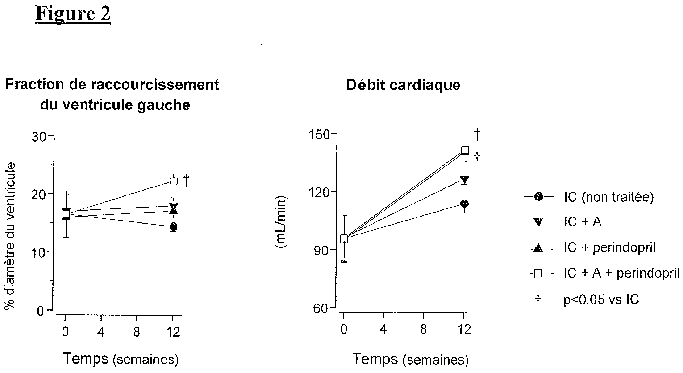

Le co-traitement par perindopril et le composé A permet de significativement augmenter la fraction de raccourcissement du ventricule gauche, c'est à dire d'améliorer sa contractilité (tableau 2 et figure 2). En conséquence, le débit cardiaque est amélioré par rapport aux animaux insuffisants cardiaques ne recevant pas de traitement.

**Tableau 2**

| | **IC (non traitée)** | **IC + A** | **IC + perindopril** | **IC + A + perindopril** |
|---|---|---|---|---|
| **fraction de raccourcissement** (% diamètre du VG) | 14,4 | 18,0 | 17,1 | 22,3^{†} |
| **débit cardiaque** (mL/min) | 114 | 127 | 142^{†} | 140^{†} |

| | | | | |
|---|---|---|---|---|
| ^{†}p<0,05 vs IC | | | | |

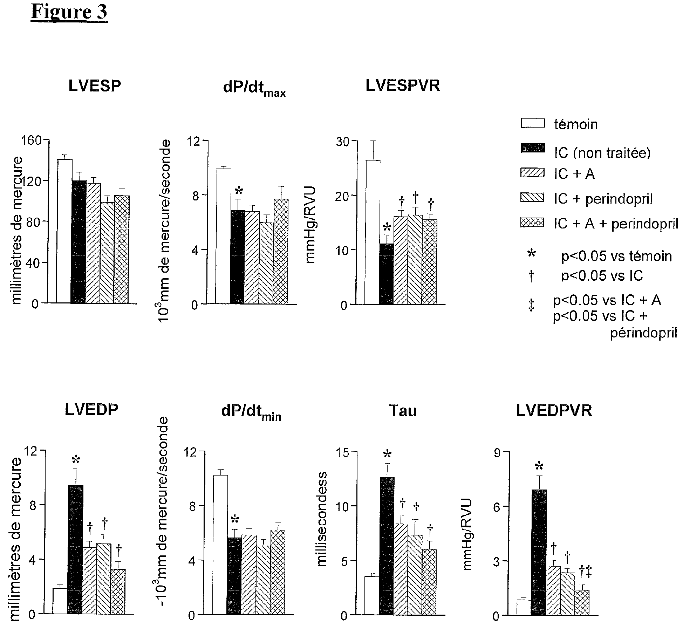

Comme le montre le tableau 3 (figure 3), les différents paramètres systoliques et diastoliques sont modifiés par l'insuffisance cardiaque. Le ventricule gauche se contracte moins bien (dP/dtₘₐₓ et LVESPVR significativement plus faibles chez les animaux IC que chez les témoins sains), ce qui dénote l'atteinte systolique. La fonction diastolique est très altérée: la pression à l'intérieur du ventricule en fin de diastole est élevée (LVEDP), le temps de relaxation (tau) est allongé et la compliance (capacité du ventricule à se distendre) est faible (LVEDPVR augmentée).

**Tableau 3**

| | **témoin** | **IC (non traitée)** | **IC + A** | **IC** + **perindopril** | **IC+A+ perindopril** |
|---|---|---|---|---|---|
| **LVESP** (mm Hg) | 140 | 120 | 118 | 99 | 105 |
| **dP/dt**ₘₐₓ (10³mm Hg/s) | 9,92 | 6,89* | 6,78 | 5,97 | 7,69 |
| **LVESPVR** (mm Hg/RVU) | 26,4 | 11,1* | 16,1^{†} | 16,4^{†} | 15,6^{†} |
| **LVEDP** (mm Hg) | 1,86 | 9,43* | 4,89^{†} | 5,17^{†} | 3,32^{†} |
| **dP/dt**ₘᵢₙ (-10³mm Hg/s) | 10,24 | 5,66* | 5,87 | 5,11 | 6,19 |
| **tau** (ms) | 3,54 | 12,64* | 8,37^{†} | 7,31^{†} | 6,05^{†} |
| **LVEDPVR** (mm Hg/RVU) | 0,84 | 6,93* | 2,70^{†} | 2,36^{†} | 1,37^{‡‡} |

| | | | | | |
|---|---|---|---|---|---|
| *p<0,05 vs témoin; ^{†}p<0,05 vs IC; ^{‡}p <0,05 vs IC+A and vs IC+perindopril | | | | | |

On constate que le traitement des animaux insuffisants cardiaques, que ce soit par perindopril seul ou par le composé A seul, améliore la fonction systolique, ce que l'on peut observer avec la LVESPVR, seul paramètre indépendant des charges.

La pression télé-diastolique et le temps de relaxation sont nettement améliorés par perindopril seul ou par le composé A seul et on note une tendance à réduire encore ces deux paramètres lorsque les deux produits sont administrés ensemble. La compliance du ventricule gauche (mesurée par la LVEDPVR), seul paramètre indépendant de la charge, est très nettement améliorée par perindopril et par le composé A. Etonnamment, cet effet est significativement augmenté lorsque les animaux reçoivent concomitamment les deux traitements.

En effet, l'association du composé A et de perindopril permet d'améliorer significativement la compliance qui revient à un seuil proche de celui des animaux témoins.

L'association de perindopril et de la N-{[(7S)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-N-méthyl- 3-oxo-1-propanamine permet donc d'améliorer la fonction diastolique altérée.

### Compositions pharmaceutiques :

### Formule de préparation pour 1000 comprimés dosés à 10 mg de composé A et 2 mg de perindopril, tert-butylamine:

| | |
|---|---|
| Composé A, fumarate | 12,48 g |
| Perindopril, *tert*-butylamine | 2 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

D'autres exemples de compositions pharmaceutiques selon l'invention sont donnés ci-dessous, à titre non limitatif :

| **Exemple** | **Composé A (mg)** | **Perindopril, sel de *tert-*butylamine (mg)** | **Perindopril, sel d'arginine (mg)** |
|---|---|---|---|
| 5 | 60 | 2 | - |
| 6 | 80 | 4 | - |
| 7 | 60 | - | 2,5 |
| 8 | 80 | - | 5 |

## Revendications

1. Association de la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-methyl-3-oxo-1-propanamine ou l'un de ses sels d'addition à un acide pharmaceutiquement acceptable, leurs hydrates ou formes cristallines, et du perindopril ou l'un de ses sels d'addition à une base pharmaceutiquement acceptable, leurs hydrates ou formes cristallines.

2. Association selon la revendication 1, **caractérisée en ce que** la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-methyl-3-oxo-1-propanamine est sous forme de chlorhydrate ou de fumarate, ou de l'un de leurs hydrates ou formes cristallines.

3. Association selon l'une des revendications 1 ou 2, **caractérisée en ce que** le perindopril est sous forme de sel de *tert*-butylamine ou d'arginine, ou de l'un de leurs hydrates ou formes cristallines.

## Patentansprüche

1. Kombination aus *N*-{[(7*S*)-3,4-Dimethoxybicyclo[4.2.0]octa-1,3,5-trien-7-yl]-methyl}-3-(7,8-dimethoxy-1,2,4,5-tetrahydro-3*H*-3-benzazepin-3-yl)-*N*-methyl-3-oxo-1-propanamin oder eines seiner Additionssalze mit einer pharmazeutisch annehmbaren Säure, deren Hydrate oder Kristallformen, und Perindopril oder eines seiner Additionssalze mit einer pharmazeutisch annehmbaren Base, deren Hydrate oder Kristallformen.

2. Kombination nach Anspruch 1, **dadurch gekennzeichnet, dass** *N*-{[(7*S*)-3,4-Dimethoxybicyclo[4.2.0]octa-1,3,5-trien-7-yl]-methyl}-3-(7,8-dimethoxy-1,2,4,5-tetrahydro-3*H*-3-benzazepin-3-yl)-*N*-methyl-3-oxo-1-propanamin in Form des Hydrochlorids oder Fumarats oder eines ihrer Hydrate oder Kristallformen vorliegt.

3. Kombination nach einem der Ansprüche 1 oder 2, **dadurch gekennzeichnet, dass** Perindopril in Form des *tert*.-Butylamin- oder Argininsalzes oder eines ihrer Hydrate oder Kristallformen vorliegt.

## Claims

1. Association of *N*-{[(7*S*)-3,4-dimethoxybicyclo[4.2.0]octa-1,3,5-trien-7-yl]methyl}-3-(7,8-dimethoxy-1,2,4,5-tetrahydro-3*H*-3-benzazepin-3-yl)-*N*-methyl-3-oxo-1-propanamine, or one of its addition salts with a pharmaceutically acceptable acid, their hydrates or crystalline forms, and perindopril, or one of its addition salts with a pharmaceutically acceptable base, their hydrates or crystalline forms.

2. Association according to claim 1, **characterised in that** the *N*-{[(7*S*)-3,4-dimethoxybicyclo[4.2.0]octa-1,3,5-trien-7-yl]methyl}-3-(7,8-dimethoxy-1,2,4,5-tetrahydro-3*H*-3-benzazepin-3-yl)-*N*-methyl-3-oxo-1-propanamine is in the form of the hydrochloride or fumarate, or one of their hydrates or crystalline forms.

3. Association according to one of claims 1 or 2, **characterised in that** the perindopril is in the form of the *tert*-butylamine or arginine salt, or one of their hydrates or crystalline forms.
